# EUROPEAN PATENT APPLICATION

(11) **EP 4 686 766 A1**
(43) Date of publication of application: **04.02.2026**
(21) Application number: 24192252.5
(22) Date of filing: 01.08.2024
(51) Int. Cl.: C12Q 1/6886

(54) **BIOMARKERS AND METHODS FOR THE EARLY DETECTION OF HEPATOCELLULAR CARCINOMA**

(71) Applicant: Siemens Healthineers AG, 91301 Forchheim (DE)
(72) Inventor: Kanne, Julian, 76829 Landau (DE); Müller-Eisert, Judith, 51503 Rösrath (DE); Zacherle, Tobias, 50668 Köln (DE)
(74) Representative: Siemens Healthineers Patent Attorneys

(57) **Abstract**

The present invention relates to a composition for diagnosing, detecting, or monitoring a liver cancer disease, comprising nucleic acid affinity ligands and/or peptide affinity ligands for a group of biomarkers comprising at least Prolactin, Alpha Fetoprotein (AFP) and Interleukin-6 (IL-6) and, optionally, further comprising one or more of Squamous Cell Carcinoma Antigen (SCC), Metallopeptidase Inhibitor 1 (TIMP-1) and CYFRA 21-1. The present invention further envisages corresponding methods for diagnosing, detecting or monitoring a cancer disease in a subject, as well as a computer-implemented method for the analysis of a sample of a subject, a computer-implemented method for providing a trained machine learning model, the use of affinity ligands for detecting, diagnosing, or monitoring a liver cancer disease, in particular early forms of liver cancer.

## Description

### TECHNICAL FIELD

The present invention is in the field of in vitro diagnostics and relates to a composition for diagnosing, detecting, or monitoring a liver cancer disease, comprising nucleic acid affinity ligands and/or peptide affinity ligands for the expression product(s) or protein(s) of a group of biomarkers comprising at least Prolactin, Alpha Fetoprotein (AFP) and Interleukin-6 (IL-6) and, optionally, further comprising nucleic acid affinity ligands and/or peptide affinity ligands for the expression product or protein of one or more of Squamous Cell Carcinoma Antigen (SCC), Metallopeptidase Inhibitor 1 (TIMP-1) and CYFRA 21-1. The present invention further envisages corresponding methods for diagnosing, detecting or monitoring a cancer disease in a subject, as well as a computer-implemented method for the analysis of a sample of a subject, a computer-implemented method for providing a trained machine learning model, the use of the nucleic acid affinity ligands and/or a peptide affinity ligands for detecting, diagnosing, or monitoring the liver cancer disease, in particular early forms of liver cancer.

### BACKGROUND

Hepatocellular carcinoma (HCC) is the most common form of liver cancer, accounting for approximately 90% of all cases. Only very limited progress has been made in the fields of diagnostics and therapy of this severe disease over the last decades. Hence, it remains an essential global health challenge and its incidence is constantly growing worldwide, with an estimated number of over >1 million individuals affected annually by 2025 (Villanueva, 2019, Hepatocellular Carcinoma. N Engl J Med;380(15), 1450-1462).

Over 90% of HCC cases occur in the setting of chronic liver disease, such as liver cirrhosis (e.g. due to chronic alcohol consumption), hepatitis B- and C- virus (HCV) infection and Non-alcoholic steatohepatitis (NASH) (Akinyemiju et al., 2017, JAMA Oncol;3(12), 1683-1691). In addition, molecular aberrations on the genetic level (mutational signatures) and tobacco exposure have been reported as potential contributors to tumorigenesis (Schulze et al., 2015, Nat Genet;47(5), 505-511). HCC remains one of the deadliest oncologic diseases with a 5-year survival rate (5-YSR) of 210. Survival rates highly depend of the stage and spread of the tumor: a localized stage is associated with a 5-YSR of 360, regional stage with a 5-YSR of 13% and a distant stage with a 5-YSR of only 3% ⁶. This implies that an early detection of HCC by adequate and sensitive tools has the potential to lead to a better survival of patients diagnosed with HCC.

Due to its development from well-characterized pre-morbidities (such as cirrhosis), HCC is most likely diagnosed through patient surveillance (Singal et al., 2020, J Hepatol; 72(2), 250-261). However, about 50% of cases are incidentally diagnosed based on imaging diagnostics caused by other reasons, such as abdominal pain, weight loss or worsening of liver dysfunction. The diagnosis of HCC is usually performed by non-invasive approaches, especially imaging. Nevertheless, imaging-based techniques harbor technical, financial and operational challenges such as problems in diagnostically discriminating pre-malignant liver diseases from HCC, a limited sensitivity of ultrasound-based surveillance, a non-general use of MRI analyses and a restricted access to care of eligible high-risk patients.

There is thus a clear need to improve the early detection of liver cancer methods, in particular through minimal-invasive tests.

### SUMMARY

The present invention addresses these needs and provides in a first aspect a composition for diagnosing, detecting, or monitoring a liver cancer disease, comprising one or more nucleic acid affinity ligands and/or one or more peptide affinity ligands for the expression product or protein of each biomarker of a group of biomarkers, the group of biomarkers comprising at least Prolactin, Alpha Fetoprotein (AFP) and Interleukin-6 (IL-6). The group may optionally, in certain embodiments, further comprise one or more nucleic acid affinity ligands and/or one or more peptide affinity ligands for the expression product or protein of one or more biomarkers selected from a group of biomarkers comprising Squamous Cell Carcinoma Antigen (SCC), Metallopeptidase Inhibitor 1 (TIMP-1) and CYFRA 21-1.

The invention is based on the finding that the mentioned biomarkers display an altered abundance in liver cancer serum samples, when compared to healthy serum samples. This panel of biomarkers provides the advantage that it can be used as standalone panel, e.g. to triage individuals for subsequent imaging-based detection methods, that are not included in the guidelines and to facilitate analysis for individuals that do not follow the regular imaging-based screening schedule. It further allows to complement positive and negative findings in a minimally invasive manner.

In a preferred embodiment, said affinity ligands comprise a set of oligonucleotides or a probe specific for an expression product of Prolactin, AFP, IL-6, SCC, TIMP-1 or CYFRA 21-1 or an antibody specific for a Prolactin, AFP, IL-6, SCC, TIMP-1 or CYFRA 21-1 protein.

In a further aspect, the present invention relates to a computer-implemented method for the analysis of a sample of a subject comprising the steps: (a) obtaining data on the levels of expression of Prolactin, AFP and IL-6 and, optionally, also of any one or more of SCC, TIMP-1 and CYFRA 21-1, in the subject's sample, preferably from a data storage; (b) inputting the obtained expression level data of step (a) into a trained machine learning model,
- wherein the machine learning model is based on a Machine learning method such as a logistic regression, linear classification or LDA, which are using a linear combination of the expression data, or Random Forest analysis, XGBoost analysis or support vector machines, and
- wherein the machine learning model is trained based on a training cohort of liver cancer patients and healthy subjects and corresponding diagnostic, prognostic and/or predictive conclusions in the context of the patients' liver cancer disease; and deriving a diagnostic, prognostic and/or predictive conclusion output with respect to the detection of the liver cancer disease in the subject.

In yet another aspect, the present invention relates to a computer-implemented method for providing a trained machine learning model for the analysis of a sample comprising the steps: (a) obtaining data on the levels of expression of Prolactin, AFP and IL-6 and, optionally, also of any one or more of SCC, TIMP-1 and CYFRA 21-1, from a cohort of liver cancer patients and healthy subjects from a data storage; (b) performing a machine learning method such as a logistic regression, linear classification, LDA, Random Forest analysis, XGBoost analysis or support vector machines using the levels of expression of Prolactin, AFP and IL-6 and, optionally, also of any one or more of SCC, TIMP-1 and CYFRA 21-1, of the cohort of step (a); and (c) training a machine learning model with the result obtained in step (b) and diagnostic, prognostic and/or predictive conclusions in the context of liver cancer corresponding to said expression, thereby obtaining a trained machine learning model. It is preferred that the machine learning method for logistic regression, linear classification or LDA is based on formula X = a_{Prolactin} · C_{Prolactin} + a_{AFP} · C_{AFP} + a_{IL-6} · C_{IL-6} + a_{SCC} · C_{SCC} + a_{TIMP-1} · C_{TIMP-1} + a_{CYFRA 21-1} · C_{CYFRA 21-1 +} a₀, wherein *aᵢ* is the to-be-learned coefficient for each marker concentration *cᵢ* and *a₀* is a standard bias coefficient.

In a preferred embodiment, the training of the machine learning model additionally includes the inputting of data of a cohort of subjects affected by liver cancer and healthy subjects concerning one or more of: age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests, as well as imaging-based data, preferably derived from an MRT, medical images and/or pathology images, or ultrasonic analysis.

In a further aspect, the present invention relates to a method of diagnosing, detecting or monitoring a liver cancer disease in a subject, comprising at least the step of determining the levels of expression of Prolactin, AFP and IL-6 and, optionally, also of any one or more of SCC, TIMP-1 and CYFRA 21-1, in a subject's sample, preferably comprising the additional step of testing in a control sample the levels of expression of Prolactin, AFP and IL-6 and, optionally, also of any one or more of SCC, TIMP-1 and CYFRA 21-1, and determining the difference of the levels of expression of Prolactin, AFP and IL-6 and, optionally, also of any one or more of SCC, TIMP-1 and CYFRA 21-1, in the subject's sample vs. the control sample.

In yet another aspect, the present invention relates to an assay for detecting, diagnosing or monitoring a liver cancer disease in a subject comprising at least the steps (a) testing in the subject's sample the levels of expression of Prolactin, AFP and IL-6 and, optionally, also of any one or more of SCC, TIMP-1 and CYFRA 21-1; (b) testing in a control sample the levels of expression of Prolactin, AFP and IL-6 and, optionally, also of any one or more of SCC, TIMP-1 and CYFRA 21-1; (c) determining the difference in expression of Prolactin, AFP and IL-6 and, optionally, also of any one or more of SCC, TIMP-1 and CYFRA 21-1, in steps (a) and (b); and (d) deciding on the presence of a liver cancer disease based on the results obtained in step (c).

In further embodiments of the methods as defined above, said methods comprise the additional step of determining the level of expression of one or more further biomarkers.

In yet other preferred embodiments, said one or more further biomarker is Alpha-Fetoprotein-L3 (AFP-L3) or protein induced by vitamin K absence/antagonist-II (PIVKA-II).

In a further embodiment, said sample is a body fluid sample, preferably a blood sample such as a serum or plasma sample, a urine sample, a urine sediment sample, a breath sample, a saliva sample, a semen sample, or a nipple aspiration sample.

It is further envisaged that the method according to the invention additionally comprises the step of evaluation of (i) the subject's imaging-based data, preferably derived from an MRT, medical images and/or pathology images, or ultrasonic analysis; and/or (ii) non-imaging-based data, such as data on age, sex, race, alcohol consumption, history of cancer, familial cancer predisposition, characteristics of cancer phenotype, histologic characteristics, histologic subtype, detectable molecular changes, preferably on the level of the genome, transcriptome, metabolome, proteome, glycome or lipidome, biomarkers and/or laboratory tests; wherein the results of the evaluation(s) contribute to the diagnostic and/or monitoring conclusion with respect to the presence of a liver cancer disease in the subject.

In a further preferred embodiment, the method further comprises the step of evaluation of the data of (i) and (ii) by a risk-predictive artificial intelligence program.

In a further aspect, the present invention relates to the use of one or more nucleic acid affinity ligands and/or one or more peptide affinity ligands with specificity for the expression product or protein of each biomarker of a group of biomarkers, the group of biomarkers comprising at least Prolactin, AFP and IL-6 and, optionally, also any one or more of SCC, TIMP-1 and CYFRA 21-1, for detecting, diagnosing, or monitoring a liver cancer disease.

In a preferred embodiment of the composition, methods or use as defined herein above, the liver cancer disease is an early form of liver cancer.

### BRIEF DESCRIPTION OF THE DRAWINGS

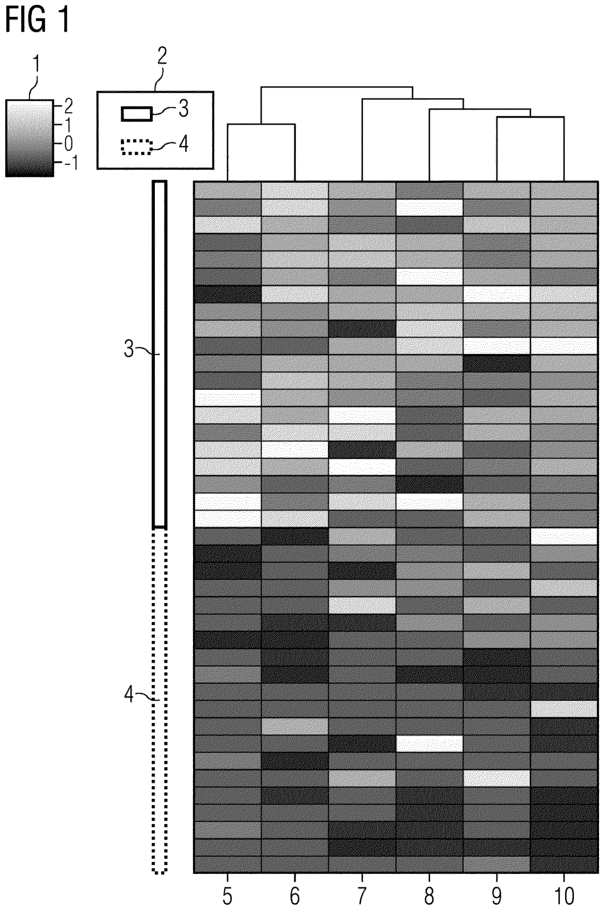

FIG. 1 shows a row clustering in the form of a heatmap of tumor and control cohorts based on protein expression levels of biomarker panel proteins according to the present invention. Protein expression levels were assessed from human serum by the Atellica Solution system. Shown is the z-score (1) in different grey scale values. The cohort type (2) is either tumor (3) or control (4). Indicated is AFP (5), Prolactin (6) TIMP-1 (7), SCC (8), CYFRA 21-1 (9) and IL-6 (**10**).

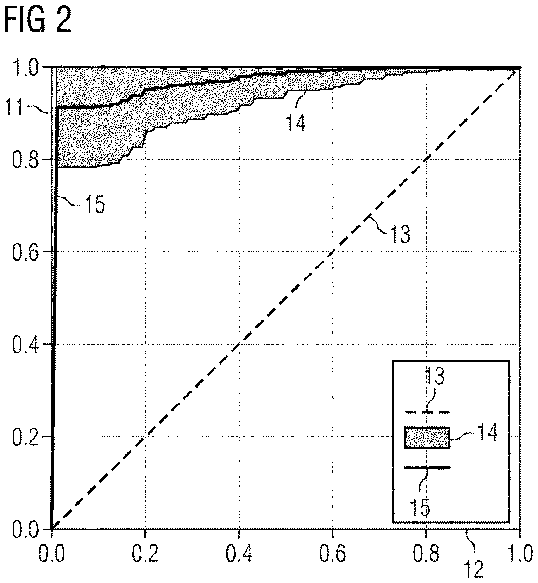

FIG. 2 shows a mean ROC curve and corresponding mean AUC values for biomarker panel proteins according to the present invention using a logistic regression classifier. An ensemble of 500 random train-test splits (70-30) was used to estimate the performance and its error. On the left-hand side, the scale of the true positive rate (**11**) is shown. On the abscissa the false positive rate (**12**) is indicated. Further indicated is random (**13**), as well as +/1 std. dev. (**14**) and the mean ROC (with AUC=0.97 +/- 0.04) (**15**).

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", "(iii)" or "(a)", "(b)", "(c)", "(d)", or "first", "second", "third" etc. and the like in the description or in the claims, are used for distinguishing between similar or structural elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e., the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a composition for diagnosing, detecting, or monitoring a liver cancer disease, the composition comprising one or more nucleic acid affinity ligands and/or one or more peptide affinity ligands for the expression product or protein of each biomarker of a group of biomarkers, the group of biomarkers comprising at least Prolactin, Alpha Fetoprotein (AFP) and Interleukin-6 (IL-6).

The term "nucleic acid affinity ligand for the Prolactin expression product" as used herein refers to a nucleic acid molecule being able to specifically bind to a Prolactin transcript, more preferably to a sequence of NCBI Reference number NM_000948.6 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000948.6 or a DNA sequence encoding an amino acid sequence being at least 90%, 91%, 92%, 93%, 94%, 95%, 96%, 97%, 98% or 99% identical to the sequence of UniProt Reference number P01236 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Prolactin protein" as used herein refers to a peptide molecule being able to specifically bind to the Prolactin protein. The peptide molecule may preferably be able to specifically bind to a protein or polypeptide comprising the amino acid sequence as set forth in UniProt Reference number P01236. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000948.6 or to a protein or polypeptide comprising an amino acid sequence being at least 90%, 91%, 92%, 93%, 94%, 95%, 96%, 97%, 98% or 99% identical to the sequence as set forth in UniProt Reference number P01236 or to any fragments of said sequences. Further envisaged are splice or modification variants to the above referenced sequences as known to the skilled person or derivable from suitable databases.

The term "nucleic acid affinity ligand for the Alpha Fetoprotein expression product" as used herein refers to a nucleic acid molecule being able to specifically bind to an Alpha Fetoprotein transcript, more preferably to a sequence of NCBI Reference number NM_001134.3 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_001134.3 or a DNA sequence encoding an amino acid sequence being at least 90%, 91%, 92%, 93%, 94%, 95%, 96%, 97%, 98% or 99% identical to the sequence of UniProt Reference number P02771 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Alpha Fetoprotein protein" as used herein refers to a peptide molecule being able to specifically bind to the Alpha Fetoprotein protein. The peptide molecule may preferably be able to specifically bind to a protein or polypeptide comprising the amino acid sequence as set forth in UniProt Reference number P02771. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_001134.3 or to a protein or polypeptide comprising an amino acid sequence being at least 90%, 91%, 92%, 93%, 94%, 95%, 96%, 97%, 98% or 99% identical to the sequence as set forth in UniProt Reference number P02771 or to any fragments of said sequences. Further envisaged are splice or modification variants to the above referenced sequences as known to the skilled person or derivable from suitable databases.

The term "nucleic acid affinity ligand for the Interleukin-6 expression product" as used herein refers to a nucleic acid molecule being able to specifically bind to an Interleukin-6 transcript, more preferably to a sequence of NCBI Reference number NM_000600.5 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_000600.5 or a DNA sequence encoding an amino acid sequence being at least 90%, 91%, 92%, 93%, 94%, 95%, 96%, 97%, 98% or 99% identical to the sequence of UniProt Reference number P05231 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Interleukin-6 protein" as used herein refers to a peptide molecule being able to specifically bind to the Interleukin-6 protein. The peptide molecule may preferably be able to specifically bind to a protein or polypeptide comprising the amino acid sequence as set forth in UniProt Reference number P05231. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000600.5 or to a protein or polypeptide comprising an amino acid sequence being at least 90%, 91%, 92%, 93%, 94%, 95%, 96%, 97%, 98% or 99% identical to the sequence as set forth in UniProt Reference number P05231 or to any fragments of said sequences. Further envisaged are splice or modification variants to the above referenced sequences as known to the skilled person or derivable from suitable databases.

The term "expression product" as used herein refers to a transcript or an mRNA molecule generated by the expression of a gene of a biomarker according to the present invention, in particular of the Prolactin, AFP and IL-6 genes. More preferably, the term relates to a processed Prolactin, AFP or IL-6 transcript. The term "peptide" refers to any type of amino acid sequence comprising more than 2 amino acids, e.g. polypeptide structures, protein structures, a protein or functional derivatives thereof. Furthermore, the peptide may, in certain embodiments, be combined with further chemical moieties or functionalities.

The term "liver cancer disease" as used herein refers to a liver cancer or uncontrolled malignant transformation and proliferation of liver tissue. The liver cancer may be a hepatocellular carcinoma (HCC). Further, the liver cancer disease may further include the presence of pre-invasive lesions or a pre-cancerous cell population, or a predisposition for cancer or tumor. In particularly preferred embodiments the liver cancer is an early form of liver cancer, e.g. a liver cancer of stage 1 or 2.

The term "detecting a liver cancer disease" as used herein means that the presence of a liver cancer disease or disorder in an organism may be determined or that such a disease or disorder may be identified in an organism. The determination or identification of a liver cancer disease or disorder may be accomplished by a comparison of the expression level of the biomarkers of the present invention with control levels. A liver cancer may be detected when the level of expression of the group of biomarkers according to the present invention comprising at least Prolactin, AFP and IL-6, is changed in comparison to a control level as defined herein above. In a specific embodiment of the present invention a liver cancer may be detected if the expression level of the group of biomarkers according to the present invention comprising at least Prolactin, AFP and IL-6 is similar to an expression level of a suitable cancer reference sample.

The term "diagnosing a liver cancer disease" as used herein means that a subject may be considered to be suffering from a liver cancer disease, e.g. early liver cancer, when the level of expression of at least Prolactin, AFP and IL-6 is changed compared to a control level, preferably if compared to a normal control level. The term "diagnosing" also refers to the conclusion reached through that comparison process.

The term "monitoring of a cancer disease" as used herein relates to the accompaniment of a diagnosed or detected liver cancer disease or disorder, e.g. early liver cancer. The accompaniment may be performed during a treatment procedure or during a certain period of time, typically during 2 months, 3 months, 4 months, 6 months, 1 year, 2 years, 3 years, 5 years, 10 years, or any other period of time. The term "accompaniment" means that states of disease, e.g. stage 1 or 2, in particular, changes of these sates of disease may be detected by comparing the level of expression of the group of biomarkers according to the present invention comprising at least Prolactin, Alpha Fetoprotein (AFP) and Interleukin-6 (IL-6) in a sample to a control level, preferably a control level of expression derived from a healthy control or to the level of expression of an established, e.g. independently established, cancer cell or cell line, e.g. a liver cancer cell line, in any type of periodical time segment, e.g. every week, every 2 weeks, every month, every 2, 3, 4, 5, 6, 7, 8, 9, 10, 11 or 12 month, every 1.5 year, every 2, 3, 4, 5, 6, 7, 8,9 or 10 years, during any period of time, e.g. during 2 weeks, 3 weeks, 1, 2, 3, 4, 5, 6, 7, 8, 9, 10, 11, 12 months, 1.5, 2, 3, 4, 5, 6, 7, 8, 9, 10, 15 or 20 years, respectively. The established, e.g. independently established, cancer cell, e.g. liver cancer cell, or cell line giving rise to an additional control level may be derived from samples corresponding to different stages of cancer development, e.g. stages 1 and 2 of the AJCC staging system. In a specific embodiment of the present invention the term relates to the accompaniment of a diagnosed liver cancer disease. The monitoring may also include the detection of the level of expression of additional genes or genetic elements, e.g. housekeeping genes such as GAPDH or PBGD etc. or comparisons with internal standards, e.g. if the analysis is performed in an automated analyzer.

In further embodiments, the composition according to the present invention additionally comprises one or more nucleic acid affinity ligands and/or one or more peptide affinity ligands for the expression product or protein of one or more biomarkers selected from a group of biomarkers comprising Squamous Cell Carcinoma Antigen (SCC), Metallopeptidase Inhibitor 1 (TIMP-1) and CYFRA 21-1. For example, the composition may comprise one or more nucleic acid affinity ligands and/or one or more peptide affinity ligands for the expression productor protein of each of Prolactin, AFP and IL-6 and any 1, 2, or 3 of SCC, TIMP-1 and CYFRA 21-1.

For example, in one embodiment, the composition may comprise a nucleic acid affinity ligand and/or a peptide affinity ligand for the expression product or protein of each of Prolactin, AFP and IL-6 and SCC.

In a further embodiment, the composition may comprise a nucleic acid affinity ligand and/or a peptide affinity ligand for the expression product or protein of each of Prolactin, AFP and IL-6 and TIMP-1.

In a further embodiment, the composition may comprise a nucleic acid affinity ligand and/or a peptide affinity ligand for the expression product or protein of each of Prolactin, AFP and IL-6 and CYFRA 21-1.

In yet another group of embodiments, the composition may comprise a nucleic acid affinity ligand and/or a peptide affinity ligand for the expression product or protein of each of Prolactin, AFP and IL-6, and SCC and TIMP-1, or of Prolactin, AFP and IL-6, and SCC and CYFRA 21-1 or of Prolactin, AFP and IL-6, and TIMP-1 and CYFRA 21-1.

In yet another group of embodiments, the composition may comprise a nucleic acid affinity ligand and/or a peptide affinity ligand for the expression product or protein of each of Prolactin, AFP and IL-6, and SCC and TIMP-1 and CYFRA 21-1.

The term "nucleic acid affinity ligand for the Squamous Cell Carcinoma Antigen (SCC) expression product" as used herein refers to a nucleic acid molecule being able to specifically bind to a SCC transcript, more preferably to a sequence of NCBI Reference number NM_006919.3 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_006919.3 or a DNA sequence encoding an amino acid sequence being at least 90%, 91%, 92%, 93%, 94%, 95%, 96%, 97%, 98% or 99% identical to the sequence of UniProt Reference number P29508 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Squamous Cell Carcinoma Antigen (SCC)" as used herein refers to a peptide molecule being able to specifically bind to the SCC protein. The peptide molecule may preferably be able to specifically bind to a protein or polypeptide comprising the amino acid sequence as set forth in UniProt Reference number P29508. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_006919.3 or to a protein or polypeptide comprising an amino acid sequence being at least 90%, 91%, 92%, 93%, 94%, 95%, 96%, 97%, 98% or 99% identical to the sequence as set forth in UniProt Reference number P29508 or to any fragments of said sequences. Further envisaged are splice or modification variants to the above referenced sequences as known to the skilled person or derivable from suitable databases.

The term "nucleic acid affinity ligand for the Metallopeptidase Inhibitor 1 (TIMP-1) expression product" as used herein refers to a nucleic acid molecule being able to specifically bind to a TIMP-1 transcript, more preferably to a sequence of NCBI Reference number NM_003254.3 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_003254.3 or a DNA sequence encoding an amino acid sequence being at least 90%, 91%, 92%, 93%, 94%, 95%, 96%, 97%, 98% or 99% identical to the sequence of UniProt Reference number P01033 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the Metallopeptidase Inhibitor 1 (TIMP-1) " as used herein refers to a peptide molecule being able to specifically bind to the TIMP-1 protein. The peptide molecule may preferably be able to specifically bind to a protein or polypeptide comprising the amino acid sequence as set forth in UniProt Reference number P01033. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_003254.3 or to a protein or polypeptide comprising an amino acid sequence being at least 90%, 91%, 92%, 93%, 94%, 95%, 96%, 97%, 98% or 99% identical to the sequence as set forth in UniProt Reference number P01033 or to any fragments of said sequences. Further envisaged are splice or modification variants to the above referenced sequences as known to the skilled person or derivable from suitable databases.

The term "nucleic acid affinity ligand for the CYFRA 21-1 expression product" as used herein refers to a nucleic acid molecule being able to specifically bind to a CYFRA 21-1 transcript, more preferably to a sequence of NCBI Reference number NM_002276.5 or a related RNA molecule. The nucleic acid affinity ligand may also be able to specifically bind to a DNA sequence being at least 90%, 91%, 92%, 93%, 94%, 95%, 96%, 97%, 98% or 99% identical to the sequence of NCBI Reference number NM_002276.5 or a DNA sequence encoding an amino acid sequence being at least 90%, 91%, 92%, 93%, 94%, 95%, 96%, 97%, 98% or 99% identical to the sequence of UniProt Reference number P08727 or to a fragment of said sequence; or be capable of amplifying a corresponding expression product, e.g. as part of a set of oligonucleotides. Further envisaged are splice or modification variants to the above referenced sequences as known to the skilled person or derivable from suitable databases.

The term "peptide affinity ligand for the CYFRA 21-1 protein" as used herein refers to a peptide molecule being able to specifically bind to the CYFRA 21-1 protein. The peptide molecule may preferably be able to specifically bind to a protein or polypeptide comprising the amino acid sequence as set forth in UniProt Reference number P08727. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_002276.5 or to a protein or polypeptide comprising an amino acid sequence being at least 90%, 91%, 92%, 93%, 94%, 95%, 96%, 97%, 98% or 99% identical to the sequence as set forth in UniProt Reference number P08727 or to any fragments of said sequences. Further envisaged are splice or modification variants to the above referenced sequences as known to the skilled person or derivable from suitable databases.

In preferred embodiments, the nucleic acid affinity ligands are or comprise a set of oligonucleotides specific for a Prolactin, an AFP and/or an IL-6 expression product, e.g. a Prolactin, AFP or IL-6 transcript or cDNA thereof. In further embodiments, the set of oligonucleotides may further additionally comprise entities which are specific for one or more of the expression products of SCC, TIMP-1 and CYFRA 21-1. In additional embodiments, the nucleic acid affinity ligand is a probe specific for a Prolactin, AFP or IL-6 expression product, e.g. transcript. In further embodiments, the nucleic acid affinity ligand may be probe specific for one or more of the expression products of SCC, TIMP-1 and CYFRA 21-1.

In yet another embodiment, the peptide affinity ligand is an antibody specific for a Prolactin, AFP or IL-6 protein. In further embodiments the peptide affinity ligand is an antibody specific for a SCC, TIMP-1 or CYFRA 21-1 protein.

In a further aspect, the present invention relates to a method of diagnosing, detecting or monitoring a liver cancer disease in a subject, comprising at least the step of determining the levels of expression of Prolactin, AFP and IL-6 and, optionally, also of any one or more of SCC, TIMP-1 and CYFRA 21-1, in a subject's sample.

The term "level of expression" as used herein refers to the amount of transcript produced during transcription or mRNA production and/or to the amount of protein or polypeptide, preferably measured as concentration of protein or polypeptide, generated by subsequent translation processes of a biomarker of the group of Prolactin, AFP, IL-6, SCC, TIMP-1 and CYFRA 21-1 as defined herein above. The present invention is thus based on the determination of the amount of nucleic acids and/or amount or, i.e. concentration, of proteins corresponding to the group of biomarkers including at least Prolactin, AFP, IL-6, and, optionally, one or more of SCC, TIMP-1 and CYFRA 21-1. In certain embodiments, only the amount of nucleic acids, e.g. transcript or RNA or cDNA derived therefrom may be determined. In other embodiments, only the amount of protein, e.g. the concentration, may be determined. In further embodiments, both, the amount of nucleic acids and proteins may be determined.

The term "sample" as used herein refers to any suitable sample type or form. Preferably, the sample is a body fluid sample, such as a blood sample including a serum or plasma sample, or a urine sample, a urine sediment sample, a breath sample, a saliva sample, a semen sample, or a nipple aspiration sample. In further embodiments, the sample may be a tissue, e.g., tissue biopsy sample. Further envisaged are liquor, and liquid biopsy samples derived from blood/urine or other body fluids. Also envisaged is to make use of previously deposited samples. In further embodiments the sample may be a cell free DNA (cfDNA) sample. The term "cfDNA" as used herein refers to degraded DNA fragments released to body fluids such as blood plasma, urine, cerebrospinal fluid, etc., wherein the cfDNA typically has a short half-life and requires rapid processing and/or stabilization. The use of cfDNA is particularly advantageous since elevated levels of cfDNA are observed in cancer.

In certain embodiments, the sample may be a tumor sample, i.e., the proteins and/or nucleic acids may be extracted from a tumor of a subject, e.g. a liver tumor. In other embodiments, the sample may be derived from a healthy subject.

The term "subject" as used herein refers to an individual such as a healthy individual that is not suspected of having a pre-invasive lesion, a pre-cancerous cell or a cancer disease; or an individual that is not suspected of having a pre-invasive lesion, a pre-cancerous cell or a cancer disease, e.g. liver cancer; or an individual that is suspected of having a pre-invasive lesion or a pre-cancerous cell; or an individual, that has a pre-invasive lesion or a pre-cancerous cell population but is not suspected of having a cancer disease, e.g. liver cancer; or an individual that has a pre-invasive lesion or a pre-cancerous cell population and is suspected of having a cancer disease, e.g. liver cancer; or an individual that is suspected of having a pre-invasive lesion or a pre-cancerous cell population; or an individual that has a pre-invasive lesion or a pre-cancerous cell population; or an individual that is suspected of having a liver cancer disease; or an individual that has a liver cancer disease. The term "patient" as used herein refers to an individual that has a pre-invasive lesion or a pre-cancerous cell population; or an individual that is afflicted by a liver cancer disease.

To "determine the level of expression" the amount of expression products as defined herein, i.e. of nucleic acids or proteins is measured. Thus, the expression levels may be determined by a method involving the detection of an mRNA or transcript encoded by any of the biomarkers, the detection of a protein encoded by any of the transcripts, preferably the concentration of the protein.

For example, the nucleic acid level may be assessed by any suitable method known to the skilled person including separation of nucleic acid molecules in agarose or polyacrylamide gels, Northern blot analysis, as well as detection via DNA arrays or microarrays. Preferably, the nucleic acid level may be detected in a quantitative RT-PCR approach, preferably in a real-time PCR approach following the reverse transcription of the mRNA transcript of the biomarkers according to the present invention. Typically, as first step, a transcript is reverse transcribed into a cDNA molecule according to any suitable method known to the person skilled in the art. A quantitative or real-time PCR approach may subsequently be carried out based on a first DNA strand obtained as described above. Also envisaged are dPCR approaches.

Preferably, a nucleic acid affinity ligand according to the present invention as defined herein above may be used.

The measurement of protein levels or concentrations of the biomarkers of the present invention or of any fragments, homologues or derivates derived thereof may be carried out via any suitable detection technique known in the art. Preferably, the protein level or concentration of the biomarkers of the present invention and derivatives thereof may be determined immunologically, e.g. by using antibodies specific for Prolactin, AFP, IL-6, SCC, TIMP-1 or CYFRA 21-1. Alternatively, antibody variants or fragments may be used.

Preferably, a peptide affinity ligand according to the present invention as defined herein above may be used.

Determination of the protein levels can, for example, be accomplished by the separation of proteins from a sample on a polyacrylamide gel, followed by identification of biomarker proteins using specifically binding antibodies in a Western blot analysis. Alternatively, proteins can be separated by two-dimensional gel electrophoresis systems. Two-dimensional gel electrophoresis is well known in the art and typically involves iso-electric focusing along a first dimension followed by SDS-PAGE electrophoresis along a second dimension. The analysis of 2D SDS-PAGE gels can be performed by determining the intensity of protein spots on the gel or can be performed using immune detection. In other embodiments, protein samples may be analyzed by mass spectroscopy. For glycosylated proteins staining and affinity-based methods may be used.

Within the context of the present invention, specific antibodies may be immobilized on a solid support. Proteins derived from samples to be analyzed may subsequently be mixed with the antibodies. A detection reaction may then be carried out, e.g. with a secondary antibody, preferably with a specific antibody.

Immunological tests which may be used in the context of the present invention, in particular for the diagnostic purposes of the present invention, include, for example, competitive and non-competitive assay systems using techniques such as Western blots, radioimmunoassay like RIA (radio-linked immunoassay), ELISA (enzyme linked immunosorbent assay), "sandwich" immunoassays, immunoprecipitation assays, precipitin reactions, gel diffusion precipitin reactions, immunodiffusion assays, agglutination assays, e.g. latex agglutination, complement-fixation assays, immunoradiometric assays, fluorescent immunoassays, e.g. FIA (fluorescence-linked immunoassay), chemiluminescence immunoassays, electrochemiluminescence immunoassay (ECLIA) and protein A immunoassays. Such assays are routine and well known to the person skilled in the art.

Furthermore, the binding affinity of an antibody to an antigen and the off-rate of an antibody-antigen interaction may be determined by competitive binding assays. One example of a competitive binding assay is a radioimmunoassay comprising the incubation of labeled antigen (e.g., ³H or ¹²⁵I) with a suitable antibody in the presence of increasing amounts of unlabeled antigen, and the detection of the antibody bound to the labeled antigen. The affinity of the antibody of interest for a particular antigen and the binding off-rates may be determined from the data by any suitable analysis approach, e.g. by a scatchard plot analysis. Competition with a second antibody may also be determined using radioimmunoassays. In this case, the antigen may be incubated with a suitable antibody conjugated to a labeled compound (e.g., ³H or ¹²⁵I) in the presence of increasing amounts of an unlabeled second antibody.

In a preferred embodiment, the method as defined above comprises the additional step of testing a control sample for the levels of expression of Prolactin, AFP, and IL-6 and, optionally, also of any one or more of SCC, TIMP-1 and CYFRA 21-1, and determining the difference of the levels of expression of Prolactin, AFP, and IL-6 and, optionally, also of any one or more of SCC, TIMP-1 and CYFRA 21-1, in the subject's sample vs. the control sample.

The term "control sample" relates to a sample from the same subject as the test sample, or to a sample derived from a different source or subject. The control sample may further be either a sample derived from the same tissue or be derived from a different tissue type. Furthermore, the testing of the subject's sample for the expression of a reference gene and the testing of a control sample may be combined. It is preferred that the control sample is the sample of a healthy subject, i.e. a subject which is not affected by a liver cancer disease. In alternative embodiments, the control sample may be a sample derived from a cancerous subject, e.g. from a subject affected by liver cancer, or from a collection of cancerous samples.

The use of control samples may, in certain embodiments, yield control levels of expression of the tested biomarkers. Accordingly, the term "control level" as used herein, relates to an expression level which may be determined at the same time and/or under similar or comparable conditions as the subject's sample by using, for example, (a) sample(s) previously collected and stored from a subject/subjects whose condition or disease state, e.g. non-cancerous, normal or healthy is/are known, e.g. have been determined by an independent molecular, histological or physiological method known to the person skilled in the art.

By comparing the results obtained by determining the levels of expression of Prolactin, AFP, or IL-6 and, optionally, also of any one or more of SCC, TIMP-1 and CYFRA 21-1, in a subject's sample and in a control sample, it may be determined whether a change or alteration of the levels of expression is given. Depending on the nature of the control sample, it may be deduced, whether the subject is affected by a liver cancer disease. For example, in case the control sample is a sample of a healthy subject, any changed level of expression of Prolactin, AFP, and IL-6 and, optionally, also of any one or more of SCC, TIMP-1 and CYFRA 21-1, in the subject's sample in comparison to the control sample is considered to be indicative of a liver cancer disease. In case the control sample is a sample of a cancerous subject, e.g. a liver cancer subject, a similar or identical levels of expression of Prolactin, AFP, and IL-6 and, optionally, also of any one or more of SCC, TIMP-1 and CYFRA 21-1, in both samples is considered to be indicative of a liver cancer disease.

The term "changed" in the context of the present invention thus denotes an increase or decrease in the expression level between a situation to be analyzed, e.g. derivable from a subject's sample, and a reference point. Expression levels are deemed to be "changed" when an expression level, e.g. in a subject's sample to be analyzed, differs by, i.e. is elevated or reduced by, for example, 5%, 6%, 7%, 8%, 9%, 10%, 15%, 20%, 25%, 30%, 40%, 50%, or more than 50% in comparison to a healthy control level, or is elevated at least 0.1 fold, at least 0.2 fold, at least 1 fold, at least 2 fold, at least 5 fold, or at least 10 fold or more in comparison to a healthy control level. If a comparison with a cancerous control level is to be carried out, an additional comparison with a healthy control level may be performed. Such an additional comparison allows for the determination of a tendency of the modification, e.g. the magnitude of an increase or decrease of the expression level may be observed and/or corresponding conclusions may be drawn.

If the expression levels of more than one biomarker are determined, e.g. of Prolactin, AFP, and IL-6 and, optionally, of any one of SCC, TIMP-1 and CYFRA 21-1, the obtained data may be statistically analyzed. For example, measured changes of expression levels may be averaged or weighed according to a suitable procedure in order to generate a summary value for the change. It is preferred that the contributions to said summary value are identical for all measured biomarkers.

In further preferred embodiments, determining of the levels of expression of Prolactin, AFP, and IL-6 and, optionally, also of any one or more of SCC, TIMP-1 and CYFRA 21-1, in a subject's sample is performed in an automatic analyzer.

In a further aspect, the present invention relates to a method for detecting, diagnosing or monitoring a cancer disease in a subject comprising at least the steps: (a) testing in a subject's sample the levels of expression of Prolactin, AFP, and IL-6 and, optionally, also of any one or more of SCC, TIMP-1 and CYFRA 21-1; (b) testing in a control sample the expression levels of Prolactin, AFP, and IL-6 and, optionally, also of any one or more of SCC, TIMP-1 and CYFRA 21-1; (c) determining the difference in expression levels of Prolactin, AFP, and IL-6 and, optionally, also of any one or more of SCC, TIMP-1 and CYFRA 21-1, in steps (a) and (b); and (d) deciding on the presence of a liver cancer disease based on the results obtained in step (c). The determination of the levels of expression may be performed as described herein above. The control sample may be a control sample as defined herein above, preferably a sample from a healthy subject.

In preferred embodiments, the testing steps in the methods as described above are based on the use of antibodies specifically binding to one of Prolactin, AFP, and IL-6 and, optionally, also to any one or more of SCC, TIMP-1 and CYFRA 21-1. Such antibodies would be known to the skilled person or can be derived from suitable literature sources.

In a further specific embodiment, the present invention relates to a method for diagnosing, detecting or monitoring a liver cancer disease in a subject, comprising: (a) determining the levels of expression of Prolactin, AFP, and IL-6 and, optionally, also of any one or more of SCC, TIMP-1 and CYFRA 21-1, in a sample of the subject; (b) determining the level of expression of a reference marker in the subject's sample or determining the level of an internal standard; (c) optionally normalizing the measured expression levels of Prolactin, AFP, and IL-6 and, optionally, also of any one or more of SCC, TIMP-1 and CYFRA 21-1, to the expression level of the reference marker or internal standard; and (d) comparing the expression levels of step (a) and step (b) and, optionally, of step (c) wherein change of the expression levels of Prolactin, AFP, and IL-6 and, optionally, also of any one or more of SCC, TIMP-1 and CYFRA 21-1, is indicative of a liver cancer disease. The determination of the levels of expression may be performed as described herein.

The term "reference marker" as used herein refers to any suitable biomarker, e.g. to a steadily expressed and continuously detectable gene, gene product, expression product, protein or protein variant in the organism of choice. The term also includes gene products such as expressed proteins, peptides, polypeptides, as well as modified variants thereof. Also encompassed are all kinds of transcripts derivable from a corresponding gene as well as modifications thereof or secondary parameters linked thereto. Alternatively or additionally, other reference parameters may also be used for reference purposes, e.g. metabolic concentrations, cell sizes etc. Typically, the reference marker is chosen as marker for which a corresponding amount of biomarkers according to the present invention may have been determined before, e.g. in cancer or non-cancer situations, or be known to the skilled person, e.g. from databases etc. Thus, there is an indirect reference to a cancerous and/or non-cancerous level of expression. In certain embodiments the reference marker is the expression product of a housekeeping gene.

The term "internal standard" as used herein refers to a protein or other type of marker, which is typically used in automatic analyzers in order to calibrate the amount, e.g. concentration of expression products or proteins. Similarly, also the internal standard may, in typical embodiments, be correlated to a corresponding amount, e.g. concentration, of biomarkers according to the present invention which may have been determined before, e.g. in cancer or non-cancer situations, or be known to the skilled person, e.g. from databases, suitable literature sources etc.

By determining the amount of a reference marker or an internal standard a calibration of the determined level of expression becomes possible. Accordingly, in certain embodiments, the measured expression levels of Prolactin, AFP, and IL-6 and, optionally, also of any one or more of SCC, TIMP-1 and CYFRA 21-1, may be normalized to the expression level of the reference marker or internal standard. The normalization may be performed according to any suitable method known to the person skilled in the art, e.g. according to normalization statistical methods like the standard z-score, Student's T-statistic, studentized residual analysis, standardized moment statistic, or coefficient variation statistic. Typically, such tests or corresponding formula, which would be known to the person skilled in the art, would be used to standardize expression data to enable differentiation between real variations in expression levels and variations due to the measurement processes. Further envisaged are algorithmic approaches, which allow for a suitable internal comparison, being equivalent to a calibration approach, as will be explained further below.

Based on the expression levels results obtained in steps (a) and (b) and optionally the normalized results obtained in step (c) a comparison of the determined expression levels is performed. Upon this comparison a change of the expression levels may be detected, e.g. as defined herein above, which would be indicative of a liver cancer disease.

It is preferred that the assay is an immunoassay such as an enzyme immunoassay (EIA), a radioimmunoassay (RUA), a fluoroimmunoassay (FIA), a chemiluminescence immunoassay (CLIA), a counting immunoassay (CIA), or an Enyzme-linked Immunosorbent Assay (ELISA). Preferably the immunoassay is performed on an analyzer.

In further specific embodiments, the testing steps in the methods as described above are specific clinical chemical testings, preferably as performed on automatic analyzers. Further information can be derived, for example, from Lee et al., 2022, Frontiers in Neurology, Vol. 13, Article 935382.

In a further group of embodiments of the methods according to the invention, said methods comprise the additional step of determining the levels of one or more further biomarkers. These additional biomarkers may be any suitable biomarkers known to the skilled person, in particular biomarkers known to be relevant in the context of liver cancer disease, more preferably in the context of an early liver cancer disease. Examples of suitable additional biomarkers include Alpha-Fetoprotein-L3 (AFP-L3) and vitamin K absence/antagonist-II (PIVKA-II). These biomarkers may be detected with suitable nucleic acid affinity ligands or peptide affinity ligands as described herein below.

For example, a peptide affinity ligand for the AFP-L3 expression product may be used. This term refers to a peptide molecule being able to specifically bind to the AFP-L3 protein. AFP-L3 is a glycoform of AFP as defined herein, that specifically binds to lens culinaris agglutinin. Further information on AFP-L3 and methods for its detection can be derived, for example, from Li et al., 2001, Clinica Chimica Acta, 313, 1-2, 15-19 or Liu et al., 2024, Communications Biology, 7, 505.

For example, a peptide affinity ligand for the PIVKA-II expression product may be used. This term refers to a peptide molecule being able to specifically bind to the PIVKA-II protein. PIVKA-II is an abnormal prothrombin containing glutamic acid (Glu) residues. In particular, when prothrombin is generated by liver cells under conditions of reduced vitamin K or in the presence of a vitamin K antagonist, the Glu residue at the N-term of the prothrombin precursor is not completely converted to γ-carboxyglutamic acid (Gla) by γ-carboxylase. Further information on PIVKA-II and methods for its detection can be derived, for example, from Tartaglione et al., 2021, PLoSOne, 16(5), e0251656.

In a further preferred embodiment of the method of diagnosing, detecting or monitoring a liver cancer disease in a subject as defined herein above, the method additionally comprises the step of evaluation of (i) the subject's imaging-based data, preferably derived from an MRT, medical images and/or pathology images, or ultrasonic analysis; and/or (ii) non-imaging-based data, such as data on age, sex, race, alcohol consumption, history of cancer, familial cancer predisposition, characteristics of cancer phenotype, histologic characteristics, histologic subtype, detectable molecular changes, preferably on the level of the genome, transcriptome, metabolome, proteome, glycome or lipidome, biomarkers and/or laboratory tests; wherein the results of the evaluation(s) contribute to the diagnostic and/or monitoring conclusion with respect to the presence of a liver cancer disease in the subject. Also envisaged is the usage of other non-invasive biomarkers such as circulating cell-free tumor DNA approaches, wherein mutations or methylation may be detected, the results of an analysis of circulating miRNAs or the results of an analysis of extracellular vesicles and metabolites.

It is particularly preferred that the evaluation of the above mentioned data is performed by a risk-predictive artificial intelligence program. For example, the method of diagnosing, detecting or monitoring a liver cancer disease in a subject as defined herein above may be combined with a method based on a trained machine learning model, e.g. as defined herein below. Also envisaged are other artificial intelligence programs which allow for the analysis of the levels of expression of Prolactin, AFP and IL-6 and, optionally, also of any one or more of SCC, TIMP-1 and CYFRA 21-1, in combination with any of the above mentioned additional data (i) and/or (ii).

In a further aspect, the present invention relates to a computer-implemented method for providing a trained machine learning model for the analysis of a sample comprising the steps: (a) obtaining data on the levels of expression of Prolactin, AFP and IL-6 and, optionally, also of any one or more of SCC, TIMP-1 and CYFRA 21-1, from a cohort of liver cancer patients and healthy subjects from a data storage; (b) performing a machine learning method such as a logistic regression, linear classification, LDA, Random Forest analysis, XGBoost analysis or support vector machines using the levels of expression of Prolactin, AFP and IL-6 and, optionally, also of any one or more of SCC, TIMP-1 and CYFRA 21-1, of the cohort of step (a); and (c) training a machine learning model with the result obtained in step (b) and diagnostic, prognostic and/or predictive conclusions in the context of liver cancer corresponding to said expression, thereby obtaining a trained machine learning model.

The method thus starts with a step of data collection on the levels of expression of Prolactin, AFP and IL-6 and, optionally, also of any one or more of SCC, TIMP-1 and CYFRA 21-1, of the samples of a cohort of liver cancer patients and healthy subjects. The term "data on the level of expression" as used herein refers to a set of data which defines the level of expression as defined herein, e.g. amount of transcript or concentration of protein of Prolactin, AFP and IL-6 and, optionally, also of any one or more of SCC, TIMP-1 and CYFRA 21-1.

In specific embodiments, the data on the levels of expression may alternatively be derived from an expression data database, e.g., a publicly available database or from literature repositories with database functions. For example, the data may be derived from the TCGA database ((https://www.cancer.gov/ccg/research/genome-sequencing/tcga), or the OncoDB (https://oncodb.org/).

The data is obtained in any suitable text-based format, e.g., as CSV, BED or XML. Preferred is the BED format.

The data may be stored locally or in a cloud system or may be derived from the data storage or database on-the-fly. The step of obtaining data may, in certain embodiments, also include an update functionality which searches for changes to levels of expression data in the database. This update may, for example, be performed within a predefined period, e.g., every week, 2, 3, 4 weeks, 2 months, 3, months, 4 months, etc.

The data may, in specific embodiments, be modified or processed, e.g., transformed into a new format, analyzed with respect to a feature of interest, or combined in any suitable way.

The levels of expression data is obtained from at least two cohorts of persons, a cohort of liver cancer patients and a cohort of healthy subjects. The cohort of liver cancer patients may preferably comprise patients affected by different types, subtypes or stages of cancer. In alternative embodiments, the cohort may also comprise patients affected by the same type, subtype or stage of liver cancer. Also envisaged are cohorts of liver cancer patients being affected by different cancer types. A very good performance of the model can be expected if the cohort of liver cancer patients and healthy subjects is of similar size. Further envisaged are cohorts which alternatively or additionally comprise patients which are comparable or similar with respect to further characteristics such as distribution of age, sex, demographic factors, alcohol consumption, ethnicity, smoking behavior, HPV infection, liver status, e.g. whether the patient is affected by fibrosis or cirrhosis, etc.

In a subsequent step, a machine learning method is performed. The machine learning method is, in preferred embodiments, a linear combination of the levels of expression of Prolactin, AFP and IL-6 and, optionally, also of any one or more of SCC, TIMP-1 and CYFRA 21-1. The term "machine learning method" as used herein refers to a any suitable method which implements an analysis of the levels of expression as defined above. In preferred embodiments, the machine learning method is a logistic regression, linear regression, LDA, Random Forest analysis, XGBoost analysis or support vector machine. It is particularly preferred that the machine learning method is a logistic regression.

In particularly preferred embodiments, the logistic regression algorithm is based on formula X = aProlactin · CProlactin + aAFP . CAFP + aIL-6 · CIL-6 + aSCC · CSCC + aTIMP-1 · CTIMP-1 + aCYFRA 21-1 . CCYFRA 21-1+ a0 wherein *aᵢ* is the to-be-learned coefficient for each marker amount or concentration *Cᵢ* and *a₀* is a standard bias coefficient. The determination of said value *Cᵢ* has been described herein above in the context of the assays of the present invention. The linear combination X of the measured expression levels data, preferably concentrations, is fed into the logistic function to obtain a value between 0 and 1. Using a suitable threshold, the samples can be classified as tumor vs. non-tumor.

In particular, the overall value of X is of importance. The coefficients *aᵢ* may be determined, for example, by using a training set, e.g. a subset of the sample cohort, which is only used for learning those coefficients such that the classification error is small within the training set. The performance metrics may then be estimated on an independent test sample set.

Logistic regression is a supervised machine learning algorithm that accomplishes binary classification tasks by predicting the probability of an outcome, event, or observation. The algorithm analyzes the relationship between one or more independent variables and classifies data into discrete classes. It is typically used in predictive modeling, where the model estimates the mathematical probability of whether an instance belongs to a specific category or not. According to the present invention the linear combination X with the trained coefficients *aᵢ* (and *a₀*) can be computed for newly measured expression level data, in particular concentrations *Cᵢ* and then fed into the logistic function to obtain a value between 0 and 1, which can be interpreted as the probability of the sample being a cancer. Choosing a classification threshold for this probability (e.g. by computing the Youden Index (Youden, 1950, Cancer, Vol. 3, Issue 1, 32-35) on the corresponding Receiver Operating Characteristic Curve allows for a final classification in tumor vs. non-tumor.

Linear classification is a data analysis technique that predicts a linear decision boundary between different higher dimensional data points.

LDA is a technique for dimensionality reduction problems as pre-processing step for pattern classification applications. The main purpose of the algorithm is the reduction of dimensions by removing redundant and dependent features from a higher dimensional space to a space with lower dimensions. Typically, LDA is used in the context of the present invention in classifier mode. The algorithm may accordingly be used to distinguish between two or more groups that are described by different variables. Further information can be derived from suitable literature sources such as Petros Xanthopoulos, Panos M. Pardalos & Theodore B. Trafalis, Robust Data Mining, Springer New York, NY, 2012, Chapter: Linear Discriminant Analysis, p27-33.

Random forest analysis is an ensemble learning method for classification, regression and other tasks that operates by constructing a multitude of decision trees at training time. For classification tasks, the output of the random forest is the class selected by most trees. For regression tasks, the mean or average prediction of the individual trees is returned. Random forests correct for decision trees' habit of overfitting to their training set. Further information can be derived from suitable literature sources such as L. Breiman, 2001, Machine Learning, Vol. 45, 5-32.

XGBoost or Extreme Gradient Boosting, is a scalable, distributed gradient-boosted decision tree (GBDT) machine learning library. It provides parallel tree boosting and is a machine learning library for regression, classification, and ranking problems. GBDT is a decision tree ensemble learning algorithm similar to random forest, for classification and regression. A model is typically build consisting of multiple decision trees. Further information can be derived from suitable literature sources such as Chen et al., 2016, Proceedings of the 22nd ACM SIGKDD International Conference on Knowledge Discovery and Data Mining, 785- 794.

A support vector machine (SVM) is a machine learning algorithm that uses supervised learning models to solve complex classification, regression, and outlier detection problems by performing optimal data transformations that determine boundaries between data points based on predefined classes, labels, or outputs. Further information can be derived from suitable literature sources such as C. Cortes, V. Vapnik, 1995, Machine Learning, Vol. 20, 273-29.

In a subsequent step of the method the machine learning models are trained based on the result obtained in previous step (b) and diagnostic, prognostic and/or predictive conclusions in the context of liver cancer corresponding to said expression, thereby obtaining a trained machine learning model. Further, by using a known health status associated to a certain sample during training allows to optimize the parameters/coefficients of the model. In general, the machine learning methods are based on the strategy to train and optimize a machine learning model and its coefficients/parameters based on a training set and then assess the performance of the machine learning model based on a test set.

The term "diagnostic, prognostic and/or predictive conclusions" as used herein refers to information on medical or therapeutic consequences of certain data on the levels of expression of Prolactin, AFP and IL-6 and, optionally, also of any one or more of SCC, TIMP-1 and CYFRA 21-1. For example, if a certain level of expression which is found in the data of the cohort of liver cancer patients (but not in the data of the cohort of healthy patients) is connected to a liver cancer disease, this connection or link may be translated into a diagnostic output, e.g., a classification as carcinogenic. Corresponding information may, for example, be derived from suitable literature sources or database entries associated with the level of expression data, but can also be derived purely based on training of the machine learning method on an annotated cohort. Similarly, if certain levels of expression are connected to early symptoms of a liver cancer disease this connection or link may be translated into a prognostic output, e.g., reflected by a classification as or potentially carcinogenic or carcinogenic in an early stage or the like. Corresponding information may, for example, be derived from suitable literature sources or database entries associated with the associated level of expression data. Also, should certain level of expression patterns be connected to therapeutic suggestions or instructions in the context of a liver cancer disease, this connection or link may be translated into a predictive output, e.g., reflected by a corresponding classification. Corresponding information may, for example, be derived from suitable literature sources or database entries associated with the level of expression data.

In a further aspect, the present invention relates to a computer-implemented method for the analysis of a sample of a subject comprising the steps: (a) obtaining data on the levels of expression of Prolactin, AFP and IL-6 and, optionally, also of any one or more of SCC, TIMP-1 and CYFRA 21-1, in the subject's sample, preferably from a data storage; (b) inputting the obtained expression level data of step (a) into a trained machine learning model wherein the machine learning model is based on a machine learning method such as a logistic regression, linear regression, LDA, Random Forest analysis, XGBoost analysis or support vector machines; and wherein the machine learning model is trained based on a training cohort of liver cancer patients and healthy subjects and corresponding diagnostic, prognostic and/or predictive conclusions in the context of the patients' liver cancer disease; and deriving a diagnostic, prognostic and/or predictive conclusion output with respect to the detection of the liver cancer disease in the subject.

Step (a) essentially corresponds to the step of obtaining expression levels data of the sample of a target subject or patient from a data storage as defined herein above. Further the trained machine learning model is a model as described herein above in detail. Accordingly, a trained machine learning model as described herein is used for the inputting step of this method. Similarly, the step (c) of the method essentially corresponds to the inference step as described in detail herein.

In a further embodiment, the present invention relates to a computer-implemented method for the analysis of a sample of a subject comprising the steps: (a) obtaining data on the levels of expression of Prolactin, AFP and IL-6 and, optionally, also of any one or more of SCC, TIMP-1 and CYFRA 21-1, from a cohort of liver cancer patients and healthy subjects from a data storage; (b) performing a machine learning method such as a logistic regression, linear regression, LDA, Random Forest analysis, XGBoost analysis or support vector machines using the levels of expression of Prolactin, AFP and IL-6 and, optionally, also of any one or more of SCC, TIMP-1 and CYFRA 21-1 of the cohort of step (a); (c) training a machine learning model with the result obtained in step (b) and diagnostic, prognostic and/or predictive conclusions in the context of liver cancer corresponding to said expression, thereby obtaining a trained machine learning model; (d) obtaining data on the levels of expression of Prolactin, AFP and IL-6 and, optionally, also of any one or more of SCC, TIMP-1 and CYFRA 21-1, from a patient's sample, preferably from a data storage; (e) inputting the obtained expression data of step (d) into the trained machine learning model of step (c); and (f) deriving a diagnostic, prognostic and/or predictive conclusion output with respect to the early detection of a liver cancer disease in the subject. The steps of this method essentially correspond to similar steps as defined in the computer-implemented method for the analysis of a sample of a subject and the computer-implemented method for providing a trained machine learning model for the analysis of a sample as defined herein. The steps of the method correspond essentially to the steps of the computer-implemented method for the analysis of a sample of a subject and the computer-implemented method for providing a trained machine learning model for the analysis of a sample as defined herein above.

In another aspect, the present invention relates to a data processing device comprising means for carrying out the computer-implemented method as defined above. The device comprises means for carrying out any one or more steps of the computer-implemented method of the present invention as mentioned herein above. Accordingly, any of the computer-implemented methods described herein may be totally or partially performed with a computer system including one or more processor(s), which can be configured to perform the steps. Accordingly, some of the present embodiments are directed to computer systems configured to perform the steps of any of the computer-implemented methods described herein, potentially with different components performing respective steps or a respective group of steps. Corresponding steps of methods may further be performed at the same time or in a different order. Additionally, portions of these steps may be used with portions of other steps from other methods. Also, all or portions of a step may be optional. Additionally, any of the steps of any of the methods can be performed with models, circuits, or other means for performing these steps.

Also envisaged is a computer program comprising instructions which, when the program is executed by a computer, cause the computer to carry out any of the computer-implemented methods of the invention as defined herein or any one or more computerizable steps of the methods of the present invention as mentioned herein.

Also envisaged is the provision of a computer-readable storage medium comprising a computer program product as defined above. The computer-readable storage medium may be connected to a server element, or be present in a cloud structure, or be connected via internet or intranet to one or more database structures, or client databases etc.

Any of the software components or computer programs or functions described herein may be implemented as software code to be executed by a processor using any suitable computer language such as, for example, Java, Python, JavaScript, VB.Net, C++, C#, C, Swift, Rust, Objective-C, Ruby, PHP, or Perl using, for example, conventional or object-oriented techniques. The software code may be stored as a series of instructions or commands on a computer readable medium for storage and/or transmission, suitable media include random access memory (RAM), a read only memory (ROM), a magnetic medium such as a hard-drive, or an optical medium such as a compact disk (CD) or DVD (digital versatile disk), flash memory, and the like. The computer readable medium may be any combination of such storage or transmission devices. Such programs may also be encoded and transmitted using carrier signals adapted for transmission via wired, optical, and/or wireless networks conforming to a variety of protocols, including the Internet. As such, a computer readable medium according to the present invention may be created using a data signal encoded with such programs. Computer readable media encoded with the program code may be packaged with a compatible device or provided separately from other devices (e.g., via internet download). Any such computer readable medium may reside on or within a single computer program product (e.g., a hard drive, a CD, or an entire computer system), and may be present on or within different computer program products within a system or network. A computer system may include a monitor, printer, or other suitable display for providing any of the results mentioned herein to a user.

In a further embodiment, the diagnostic and/or monitoring conclusion with respect to the presence of a liver cancer disease in the subject may additionally be based on the evaluation of further data. It is particularly preferred that within the computer-implemented methods as described herein, the training of the machine learning model additionally includes the inputting of data of a cohort of subjects affected by liver cancer and healthy subjects concerning one or more of: age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests, as well as imaging-based data, preferably derived from an MRT, medical images and/or pathology images, or ultrasonic analysis.

In a further aspect, the present invention relates to the use of one or more nucleic acid affinity ligands and/or one or more peptide affinity ligands with specificity for the expression products or proteins of a group of biomarkers, the group of biomarkers comprising at least Prolactin, AFP, and IL-6 and, optionally, also any one or more of SCC, TIMP-1 and CYFRA 21-1, for detecting, diagnosing, or monitoring a liver cancer disease. The nucleic acid affinity ligands and peptide affinity ligands for the expression products or proteins correspond to those defined herein above. Further contemplated is the use of the biomarkers Prolactin, AFP, and IL-6 for diagnosing, detecting, or monitoring a liver cancer disease. Also envisaged is the use of the biomarkers Prolactin, AFP, and IL-6 and any one or more of SCC, TIMP-1 and CYFRA 21-1 for diagnosing, detecting, or monitoring a liver cancer disease.

The following figures and example are provided for illustrative purposes. It is thus understood that the figures and example are not to be construed as limiting. The skilled person in the art will clearly be able to envisage further modifications of the principles laid out herein.

### EXAMPLES

### LIVER CANCER DETECTION PROCEDURE

### 1. Principles of the procedure

All samples were processed on the Atellica IM analyzer (Siemens Healthineers Diagnostics Inc.). The Atellica IM assays are 2-site sandwich assays (PRL, AFP, TIMP-1, SCC, CYFRA) or a one-step direct immunoassay (IL-6) using chemiluminescence technology. The assays utilize acridinium ester-labelled, analyte-specific antibodies directed against the respective target as the Lite Reagent. The solid phase consists of antibody-coated paramagnetic microparticles. A direct relationship exists between the amount of biomarker present in the patient sample and the amount of relative light units (RLUs) detected by the system.

### 2. Measurement Ranges

The measurement ranges were as follows:

| | |
|---|---|
| Prolactin: | 0,30-200 ng/mL |
| AFP | 1.3-1000.0 ng/mL |
| IL-6 | 2.7-5500.0 pg/mL |
| SCC | 0.24-70.00 ng/mL |
| TIMP-1 | 3.5-1300.0 ng/mL |
| CYFRA 21-1 (Cytokeratin-19): | 0,50-100 ng/mL |

### 3. Sample Handling

Serum was generated from 5 ml blood that was centrifuged at 2000g for 10 min at 4°C and stored at -80°C. In total, up to 20 serum samples from HCC patients and 20 serum samples from healthy donors were analyzed. Healthy control samples were purchased from Milan Analytics, Discovery Life Science and Precision Medicine. HCC samples were provided by Indivumed. All samples were processed according to the Instructions for Use (IFU) for each assay when analyzing patient specimen to ensure all sample and handling limitations are accounted for, and sufficient volume is available for testing. As the samples were frozen, they were thawed while gently mixed on an orbital mixer for 30 minutes and briefly vortexed. Before placing the samples on the system, it was ensured that samples were free of bubbles or foam and showed traces of fibrin or other particular matter.

Before loading the samples on the Atellica instrument system, the assay specific master curve and test definition values were entered by scanning the 2D barcodes.

Before loading the reagents on the system, they were properly mixed and visually inspected to make sure all particles are resuspended.

The system was equipped with sufficient reagent packs loaded in the reagent compartment. When using assays with automated dilutions, ensure that appropriate diluent is loaded in the reagent compartment.

For calibration of each assay, the appropriate calibrator materials for in accordance with the calibrator's instructions for use were applied.

For quality control, the appropriate quality control materials of known analyte concentrations of at least 2 levels (low and high) were analyzed at least once during each day that samples were analyzed. The quality control material was used in accordance with the IFU. A satisfactory level of performance is achieved when the analyte values obtained are within the expected control interval for the system or within your interval, as determined by an appropriate internal laboratory quality control scheme.

Details of the principles of the assay's procedure and description of the automated steps were followed according to the respective IFU.

## Claims

1. A composition for diagnosing, detecting, or monitoring a liver cancer disease, comprising one or more nucleic acid affinity ligands and/or one or more peptide affinity ligands for the expression product or protein of each biomarker of a group of biomarkers, the group of biomarkers comprising at least Prolactin, Alpha Fetoprotein (AFP) and Interleukin-6 (IL-6).

2. The composition of claim 1, additionally comprising one or more nucleic acid affinity ligands and/or one or more peptide affinity ligands for the expression product or protein of one or more biomarkers selected from a group of biomarkers comprising Squamous Cell Carcinoma Antigen (SCC), Metallopeptidase Inhibitor 1 (TIMP-1) and CYFRA 21-1.

3. The composition of claims 1 or 2, wherein said affinity ligands comprise a set of oligonucleotides or a probe specific for an expression product of Prolactin, AFP, IL-6, SCC, TIMP-1 or CYFRA 21-1, or an antibody specific for a Prolactin, AFP, IL-6, SCC, TIMP-1 or CYFRA 21-1 protein.

4. A computer-implemented method for the analysis of a sample of a subject comprising the steps:
(a) obtaining data on the levels of expression of Prolactin, AFP and IL-6 and, optionally, also of any one or more of SCC, TIMP-1 and CYFRA 21-1, in the subject's sample, preferably from a data storage;
(b) inputting the obtained expression level data of step (a) into a trained machine learning model,
- wherein the machine learning model is based on a machine learning method such as a logistic regression, linear regression, LDA, Random Forest analysis, XGBoost analysis or support vector machines using the expression data, and
- wherein the machine learning model is trained based on a training cohort of liver cancer patients and healthy subjects and corresponding diagnostic, prognostic and/or predictive conclusions in the context of the patients' liver cancer disease; and
(c) deriving a diagnostic, prognostic and/or predictive conclusion output with respect to the detection of the liver cancer disease in the subject.

5. A computer-implemented method for providing a trained machine learning model for the analysis of a sample comprising the steps:
(a) obtaining data on the levels of expression of Prolactin, AFP and IL-6 and, optionally, also of any one or more of SCC, TIMP-1 and CYFRA 21-1, from a cohort of liver cancer patients and healthy subjects from a data storage;
(b) performing a machine learning method such as a logistic regression, linear regression, LDA, Random Forest analysis, XGBoost analysis or support vector machines using a linear combination of the levels of expression of Prolactin, AFP and IL-6 and, optionally, also of any one or more of SCC, TIMP-1 and CYFRA 21-1, of the cohort of step (a), preferably based on formula X = a_{Prolactin} · C_{Prolactin} + a_{AFP} · C_{AFP} + a_{IL-6} · C_{IL-6} + a_{SCC} · C_{SCC} + a_{TIMP-1} · C_{TIMP-1} + a_{CYFRA 21-1} · C_{CYFRA 21-1 +} a₀, wherein *aᵢ* is the to-be-learned coefficient for each marker concentration *cᵢ* and *a₀* is a standard bias coefficient;
(c) training a machine learning model with the result obtained in step (b) and diagnostic, prognostic and/or predictive conclusions in the context of liver cancer corresponding to said expression, thereby obtaining a trained machine learning model.

6. The computer-implemented method of claims 4 or 5, wherein the training of the machine learning model additionally includes the inputting of data of a cohort of subjects affected by liver cancer and healthy subjects concerning one or more of: age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests, as well as imaging-based data, preferably derived from an MRT, medical images and/or pathology images, or ultrasonic analysis.

7. A method of diagnosing, detecting or monitoring a liver cancer disease in a subject, comprising at least the step of determining the levels of expression of Prolactin, AFP and IL-6 and, optionally, also of any one or more of SCC, TIMP-1 and CYFRA 21-1, in a subject's sample, preferably comprising the additional step of testing a control sample for the levels of expression of Prolactin, AFP and IL-6 and, optionally, also of any one or more of SCC, TIMP-1 and CYFRA 21-1, and determining the difference of the levels of expression of Prolactin, AFP and IL-6 and, optionally, also of any one or more of SCC, TIMP-1 and CYFRA 21-1, in the subject's sample vs. the control sample.

8. A method for detecting, diagnosing or monitoring a liver cancer disease in a subject comprising at least the steps:
(a) testing in the subject's sample the levels of expression of Prolactin, AFP and IL-6 and, optionally, also of any one or more of SCC, TIMP-1 and CYFRA 21-1;
(b) testing in a control sample the expression levels of Prolactin, AFP and IL-6 and, optionally, also of any one or more of SCC, TIMP-1 and CYFRA 21-1;
(c) determining the difference in the expression levels of Prolactin, AFP and IL-6 and, optionally, also of any one or more of SCC, TIMP-1 and CYFRA 21-1, tested in steps (a) and (b); and
(d) deciding on the presence of a liver cancer disease based on the results obtained in step (c) .

9. The computer-implemented method of claims 4, 5 or 6, or the method of claims 7 or 8, wherein additionally the level of expression of one or more further biomarkers is determined.

10. The computer-implemented method or method of claim 9, wherein said one or more further biomarker is Alpha-Fetoprotein-L3 (AFP-L3) or protein induced by vitamin K absence/antagonist-II (PIVKA-II).

11. The method of any one of claims 4 to 10, wherein said sample is a body fluid sample, preferably a blood sample such as a serum or plasma sample, a urine sample, a urine sediment sample, a breath sample, a saliva sample, a semen sample, or a nipple aspiration sample.

12. The method of any one of claims 7 and 9 to 11, additionally comprising the step of evaluation of
(i) the subject's imaging-based data, preferably derived from an MRT, medical images and/or pathology images, or ultrasonic analysis; and/or
(ii) non-imaging-based data, such as data on age, sex, race, alcohol consumption, history of cancer, familial cancer predisposition, characteristics of cancer phenotype, histologic characteristics, histologic subtype, detectable molecular changes, preferably on the level of the genome, transcriptome, metabolome, proteome, glycome or lipidome, biomarkers and/or laboratory tests;
wherein the results of the evaluations contribute to the diagnostic and/or monitoring conclusion with respect to the presence of a liver cancer disease in the subject.

13. The method of claim 12, further comprising the step of evaluation of the data of (i) and (ii) by a risk-predictive artificial intelligence program.

14. Use of one or more nucleic acid affinity ligands and/or one or more peptide affinity ligands with specificity for the expression product or protein of each biomarker of a group of biomarkers, the group of biomarkers comprising at least Prolactin, AFP and IL-6 and, optionally, also any one or more of SCC, TIMP-1 and CYFRA 21-1, for detecting, diagnosing, or monitoring a liver cancer disease.

15. The composition of claims 1, 2 or 3, the method of any one of claims 4 to 13, or the use of claim 14, wherein said liver cancer disease is an early form of liver cancer.
